# EUROPEAN PATENT SPECIFICATION

(11) **EP 0 671 898 B1**
(45) Date of publication and mention of the grant of the patent: **05.11.1997**
(21) Application number: 94900953.4
(22) Date of filing: 30.11.1993
(51) Int. Cl.: A61F 13/00

(54) **BANDAGE**
BANDAGE
BANDAGE

(30) Priority: 01.12.1992 GB 9225146
(43) Date of publication of application: 20.09.1995
(73) Proprietor: ConvaTec Limited, Clwyd CH5 2NU (GB)
(72) Inventor: ALLEN, Kenneth Alfred, Nottingham NG15 6LR (GB)
(74) Representative: Jones, Stephen Anthony
(86) International application number: GB9302469
(87) International publication number: WO9412133

(56) References cited:
- EP-A- 0 475 811
- DE-A- 3 640 979
- FR-A- 2 499 416
- US-A- 3 613 679

## Description

This invention relates to bandages, in particular to elasticated bandages of the type which have to be applied in such a way as to exert a pre-determined sub-bandage pressure.

Bandages applied to certain wounds, eg leg ulcers, have to be applied in such a way as to exert a pre-determined sub-bandage pressure. The optimum pressure will be determined by medical or nursing staff according to the nature and severity of the wound. The bandages which are applied are elasticated and the pressure exerted is a function of the degree of extension of the bandage and the circumference of the limb to which it is applied. For one commercially available bandage, for example, the following table is published:

**Table I**

| Extension | Pressure exerted / mm Hg | | |
|---|---|---|---|
| | Limb circumference / cm | | |
| | 18-26 | 27-35 | 36-50 |
| 50% | 36-25 | 24-18 | |
| 70% | 50-37 | 37-28 | 28-19 |

In use, a nurse faced with the task of applying such a bandage must first measure or estimate the circumference of the patient's limb and then apply the bandage with the correct degree of extension to achieve the prescribed pressure. This is a difficult operation to perform with any degree of accuracy, with the result that incorrect pressure is often achieved. This may cause sub-optimal healing, or discomfort for the patient.

In an attempt to overcome this problem, it has been proposed (see Journal of Wound Care, Sept/Oct 1992, page 23 onwards) to print on a bandage a visual aid in the form of similar rectangles which, when the bandage is stretched to a predetermined extension, take the shape of squares. A disadvantage of this arrangement is that the nurse must still estimate the circumference of the limb and then try to choose the extension which will give rise to the required sub-bandage pressure for that limb. Since the extension at which the rectangles become square will only be appropriate for one limb size, the visual aid is of limited utility.

A further disadvantage of this arrangement is that the printing of the pattern on the bandage results in a non-uniform, non-smooth surface having "high spots" which may give rise to irritation of the wound or other harmful effects. There is also a risk that the printing ink or marker substance may give rise to allergic reactions with some patients, which cannot be predicted. The problem is particularly acute, since the printing ink is generally inflexible and may therefore crack when the bandage is extended and particles of ink may become dislodged, thereby contaminating the wound.

FR-A-2499416 discloses a bandage which bears two rows of rectangles which are dimensioned such that they are transformed into squares by stretching of the bandage to two different extents.

There has now been devised an improved form of elasticated bandage which overcomes or substantially mitigates the above-mentioned disadvantages.

According to the invention, there is provided an elasticated bandage bearing a geometrical pattern comprising two components which adopt recognisable configurations when the bandage is extended to corresponding pre-determined degrees, characterised in that said two components are alternating large and small rectangles having short sides which lie parallel to the longitudinal axis of the bandage and longer sides which lie transverse to the bandage, the short sides of the large and small rectangles being colinear and together forming an application guideline located half-way between the bandage longitudinal edges.

The bandage according to the invention is advantageous in that it provides a direct visual indication of the attainment of a particular extension (and hence of a certain tension in the bandage, giving rise to a certain sub-bandage pressure), without the need for a quantitative estimation of the limb circumference.

It is particular preferred that the different degrees of extension at which the two components attain their recognisable configurations should correspond to the bandage tensions most commonly required in practice. We have found that tensions generated in the bandage by exerting a force of 1N and 1.5N across the width of the bandage are suitable for a wide range of limb sizes. In a preferred embodiment, therefore, the two components of the pattern adopt their recognisable configuration when the bandage tension corresponds to a force exerted across the width of the bandage of 1N and 1.5N respectively. The lower tension is appropriate for smaller limbs, and the higher tension for larger limbs. A nurse applying the bandage need only make a qualitative assessment of the limb size as being either "large" or "small" and extend the bandage until the respective recognisable configuration is attained.

Preferably, the bandage when applied to a limb gives rise to a pressure at the ankle of at least 40mm Hg.

The geometrical pattern borne by the bandage comprises rectangles, the short sides of which lie parallel to the longitudinal axis of the bandage, and the longer sides transverse to the bandage. Extension of the bandage along its long axis will elongate the short sides of the rectangle. A point is reached at which the short sides are the same length as the long sides. In this condition, the rectangle has become a square which is recognisable as such and thus indicates that the required extension has been reached.

Experience has shown that the point at which the rectangle becomes a square is readily detectable by the user, and achieving substantially the requisite degree of tension is reproducible. If the requisite tension is attained at 50% extension, the ratio between the lengths of the long and short sides of the rectangle should be 3:2 in the unstretched condition. If the requisite tension is attained at 75% extension, the ratio should be 7:4.

The rectangles may be discrete or may be linked.

The large and small rectangles are arranged with colinear short edges. Together, the colinear short edges form a continuous line running the length of the bandage. The said line is located half-way between the bandage longitudinal edges, and performs the function of the central application guide line conventionally present on such bandages. This application guide line is important since the sub-bandage pressure depends on the degree of overlap with which the bandage is applied, and 50% overlap is conventionally used.

Although two components in the pattern are generally sufficient for practical purposes, eg to indicate the two extremes of an operating range or two pre-determined tensions, it is of course possible for further components to be provided. One example would be rectangles of three different proportions, indicating three different pre-determined tensions.

The geometrical pattern may be printed on the bandage. However, it is preferred that the pattern should be part of the structure of the fabric ie it should be knitted or woven into the fabric. This prevents any real or perceived differences in the thickness or feel of the bandage, and eliminates the problem of physiological reactions to the printing ink used.

In colour, the bandage will generally be white or flesh-coloured. Most preferably, the geometrical pattern is yellow or orange since this colour is easily visible on such a light background and is also easily seen by persons who suffer from colour-blindness.

Apart from the provision of the geometrical pattern, the bandage of the present invention may be generally conventional in construction, and may be manufactured using techniques, eg machine knitting, which are conventionally used for the manufacture of elasticated bandages. In line with conventional machine-knitting practice, the pattern may be formed using pattern bars which are driven to reciprocate by suitable camming arrangements.

The invention will now be described in greater detail, by way of illustration only, with reference to the accompanying drawings in which
Figure 1 is a plan view of part of an elasticated bandage according to the presently preferred embodiment of the invention in the unstretched condition,
Figure 2 shows the bandage of Figure 1 extended to a first pre-determined tension, and
Figure 3 shows the bandage of Figure 1 extended to a second greater pre-determined tension.

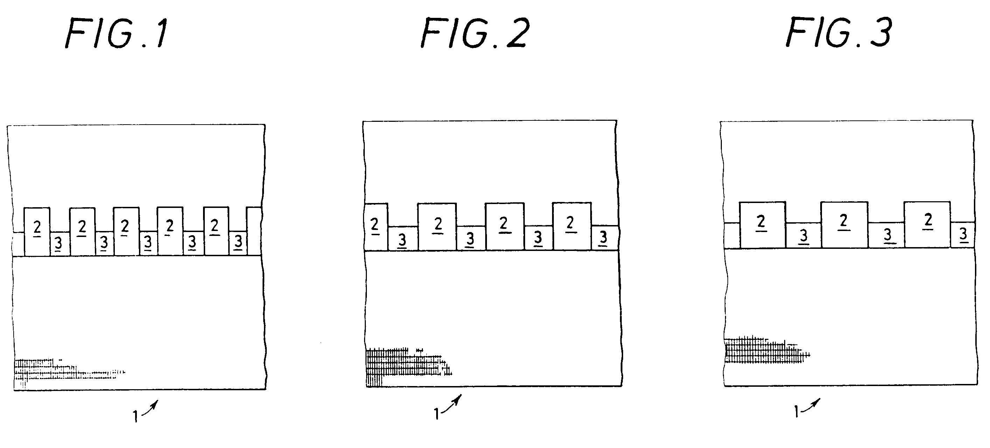

Referring first to Figure 1, an elasticated bandage (generally designated 1) comprises a band approximately 10cm wide of elasticated knitted fabric. The preferred bandage is made from a

Lycra elastomer, a cotton-viscose blend yarn, and nylon. Knitted into the structure of the bandage (1), longitudinally of the bandage, is a geometrical pattern comprising a line of alternating small and large rectangles (2,3 respectively). The longer sides of both small and large rectangles (2,3) are arranged transverse to the longitudinal axis of the bandage (1), the shorter sides being parallel to that axis. The pattern of rectangles (2,3) is knitted in yellow yarn, the remainder of the fabric band being "off-white", ie flesh-coloured.

The smaller rectangles (2) measure 11mm x 7.5mm; the larger rectangles (3) 21mm x 12mm. When the bandage (1) is stretched, the shorter sides of the rectangles (2,3) become longer. When the bandage is stretched to the first predetermined tension (as shown in Figure 2), which in this case occurs at 50% extension, the longer and shorter sides of the smaller rectangles (2) become approximately equal in length and the small rectangles (2) become squares. Similarly, when the bandage is stretched to a second, greater pre-determined tension, in this case occurring at 75% extension, the larger rectangles (3) become square, as shown in Figure 3.

The first pre-determined tension is generated by exerting a force across the width of the bandage of approximately 1N. At this tension, the sub-bandage pressure for a patient with a relatively small limb circumference is about 45mm Hg (6.0kPa). Hence, for such a patient, to apply a compressive force of 45mm Hg (6.0kPa), the bandage (1) is stretched until the small rectangles (2) become square. For a higher compressive force, say of 68mm Hg (9.1 kPa), the bandage is stretched until the larger rectangles (3) become square, ie to a tension generated by exerting a force across the width of the bandage of 1.5N. For an intermediate force the bandage would be stretched to a degree between these two extremes, ie beyond the point at which the smaller rectangles (2) become square, but before the larger rectangles (3) become square. For a patient with a relatively large limb circumference, a sub-bandage pressure of about 45mm Hg (6.0kPa) is achieved at the second pre-determined tension, ie at the extension at which the larger rectangles (3) become square.

In the known bandage in which a pattern is printed on the bandage material, the recognition of the rectangles changing to squares is provided to assure the bandager that the extension achieved is a desired amount, eg 50%. In contrast, in the present invention as particularly described the recognition of the rectangles taking up square configurations assures the bandager that the right amount of tension and thus a suitable sub-bandage pressure, eg 40mm Hg (5.3 kPa) of above has been applied. Only a qualitative estimation of the limb circumference is required.

## Claims

1. An elasticated bandage bearing a geometrical pattern comprising two components which adopt recognisable configurations when the bandage is extended to corresponding pre-determined degrees, characterised in that said two components are alternating large and small rectangles having short sides which lie parallel to the longitudinal axis of the bandage and longer sides which lie transverse to the bandage, the short sides of the large and small rectangles being colinear and together forming an application guideline located half-way between the bandage longitudinal edges.

2. A bandage as claimed in claim 1, wherein the degrees of extension at which the two components attain their recognisable configurations correspond to tensions generated in the bandage by exerting a force of approximately 1N and 1.5N across the width of the bandage.

3. A bandage as claimed in claim 1 or claim 2, wherein the bandage when applied to a limb gives rise to a pressure at the ankle of at least 40mm Hg (5.3kPa).

4. A bandage as claimed in any preceding claim, wherein the rectangles are linked.

5. A bandage as claimed in any preceding claim, wherein the bandage is white or flesh-coloured, and the geometrical pattern is yellow or orange.

6. A bandage as claimed in any preceding claim, wherein the pattern is knitted into the structure of the fabric.

7. A bandage as claimed in claim 6, wherein the geometrical pattern is formed with a yarn of a colour contrasting to that of the remainder of the bandage.

## Patentansprüche

1. Elastische Bandage, die ein geometrisches Muster trägt, das zwei Komponenten umfasst, die erkennbare Aufbauten annehmen, wenn die Bandage auf entsprechende vorbestimmte Grade ausgedehnt wird, dadurch gekenzeichnet, dass die beiden Komponenten sich abwechslende grosse und kleine Rechtecke mit kurzen Seiten sind, die parallel zu der Längsachse der Bandage liegen und längeren Seiten, die quer zu der Bandage liegen, wobei die kurzen Seiten der grossen und kleinen Rechtecke miteinander geradlinig sind und zusammen eine Aufbringungsrichtlinie bilden, die auf halbem Weg zwischen den Längskanten der Bandage angeordnet sind.

2. Bandage nach Anspruch 1, in der die Grade der Ausdehnung, bei der die beiden Komponenten ihre erkennbaren Aufbauten erreichen, Spannungen entsprechen, die in der Bandage durch Ausüben einer Kraft vin ungefähr 1N und 1,5N über der Breite der Bandage erzeugt werden.

3. Bandage nach Anspruch 1 oder Anspruch 2, in der die Bandage, wenn sie auf ein Körperglied aufgebracht wird einen Druck von wenigstens 40mm HG (5,3kPa) auf dem Knöchel hervorruft.

4. Bandage nach einem der vorhergehenden Ansprüche, in der die Rechtecke verbunden sind.

5. Bandage nach einen der vorhergehenden Ansprüche, in der die Bandage weiss oder fleischfarben ist, und das geometrische Muster gelb oder orange ist.

6. Bandage nach einem der vorhergehenden Ansprüche, in der das Muster in die Struktur des Stoffs gestrickt ist.

7. Bandage nach Anspruch 6, in der das geometrische Muster mit einein Garn aus einer Farbe gebildet ist, das im Kontrast zu dem Rest der Bandage steht.

## Revendications

1. Bande élastique de pansement portant un motif géométrique comportant deux éléments permettant d'adopter des configurations reconnaissables lors que la bande de pansement est allongée selon des longueurs prédéterminées, caractérisée en ce que lesdits deux éléments représentent en alternance des petits et de grands rectangles ayant les côtés courts situés parallèles avec l'axe longitudinal de la bande de bande de pansement et les longs côtés situés en sens transversal de la bande, les côtés courts des grands et petits rectangles étant co-linéaires et formant ensemble un guide pour la pose se situant à mi-parcours entre les bords longitudinaux de la bande de pansement.

2. Bande de pansement tel que revendiquée à la revendication 1, dont les longueurs d'extension auxquelles les deux éléments rejoignent leurs configurations reconnaissables correspondent aux tensions générées dans la bande suite à l'application d'une force de traction d'environ 1 et 1,5N en travers sur la largeur de la bande de pansement.

3. Bande de pansement tel que revendiquée à la revendication 1 ou à la revendication 2, selon lesquelles la bande posée sur un membre donne lieu à une pression à la cheville d'un minimum de 40mm Hg (5,3 kPa).

4. Bande de pansement tel que revendiquée à l'une ou l'autre des revendications précédentes, dont les rectangles sont reliés l'un à l'autre.

5. Bande de pansement tel que revendiquée à l'une ou l'autre des revendications précédentes, dont la bande est de couleur blanche ou rose-chair et le motif géométrique est jaune ou orange.

6. Bande de pansement tel que revendiquée à l'une ou l'autre des revendications précédentes, dont le motif est tricoté dans la structure du tissu.

7. Bande de pansement tel que revendiquée à la revendication 6, dont le motif géométrique est formé au moyen d'un fil de couleur contrastante par rapport au reste de la bande.
